(19) **Europäisches Patentamt**

**European Patent Office**

**Office européen des brevets**

(11) **EP 4 368 253 A1**

(12) **EUROPEAN PATENT APPLICATION**

| | |
|---|---|
| (43) Date of publication:<br>**15.05.2024 Bulletin 2024/20** | (51) International Patent Classification (IPC):<br>***A61P 43/00*** *(2006.01)* ***C07K 14/195*** *(2006.01)* |
| (21) Application number: **22206059.2** | (52) Cooperative Patent Classification (CPC):<br>**A61P 43/00; A61K 38/164; C07K 14/195** |
| (22) Date of filing: **08.11.2022** | |

| | |
|---|---|
| (84) Designated Contracting States:<br>**AL AT BE BG CH CY CZ DE DK EE ES FI FR GB GR HR HU IE IS IT LI LT LU LV MC ME MK MT NL NO PL PT RO RS SE SI SK SM TR**<br>Designated Extension States:<br>**BA**<br>Designated Validation States:<br>**KH MA MD TN** | (72) Inventors:<br>• **Skokowa, Julia**<br>  **72127 Kusterdingen (DE)**<br>• **ElGamacy, Mohammad**<br>  **72076 Tübingen (DE)** |
| (71) Applicants:<br>• **Eberhard Karls Universität Tübingen, Medizinische Fakultät**<br>  **72074 Tübingen (DE)**<br>• **Max-Planck-Gesellschaft zur Förderung der Wissenschaften e.V.**<br>  **80539 München (DE)** | (74) Representative: **Witte, Weller & Partner Patentanwälte mbB**<br>  **Postfach 10 54 62**<br>  **70047 Stuttgart (DE)**<br><br>Remarks:<br>The complete document including Reference Table(s) and the Sequence Listing(s) can be downloaded from the EPO website |

(54) **METAL-BINDING POLYPEPTIDE**

(57) The present invention relates to a polypeptide for use as a metal-binder, a protein comprising said polypeptide, a nucleic acid molecule encoding said polypeptide or protein, an expression vector comprising the nucleic acid molecule, a recombinant host cell comprising said polypeptide, protein, nucleic acid molecule and/or expression vector, a pharmaceutical composition comprising the said polypeptide, protein, nucleic acid molecule, expression vector and/or host cell, and to a kit.

**EP 4 368 253 A1**

**Description**

The present invention relates to a polypeptide for use as a metal-binder, a protein comprising said polypeptide, a nucleic acid molecule encoding said polypeptide or protein, an expression vector comprising the nucleic acid molecule, a recombinant host cell comprising said polypeptide, protein, nucleic acid molecule and/or expression vector, a pharmaceutical composition comprising the said polypeptide, protein, nucleic acid molecule, expression vector and/or host cell, and to a kit.

The present invention in particular relates to several novel peptide sequences derived from wild-type Csp1 protein, characterized by high-affinity and high-capacity metal-binding properties.

BACKGROUND OF THE INVENTION

The functional diversity of proteins is often expanded by their capacity to interact with and structurally incorporate other chemical moieties beyond the proteinogenic amino acids, such as post-translational modifications, and binding to ligands and metals. Particularly, metal-binding proteins serve the essential functions including catalysis, sensing, transport, and storage; see Malmstrom and Neilands (1964), Metalloproteins. Annual Review of Biochemistry, 33(1): p. 331-354. Designer metalloproteins can be tailored to encode one or more of such functions; see Lu et al. (2009), Design of functional metalloproteins. Nature, 460(7257): p. 855-862, and Chalkley et al. (2022), De novo metalloprotein design. Nature Reviews Chemistry 6(1): p. 31-50.

The combined design objective of engineering proteins capable of high-affinity metal binding, efficient storage, and transport, can result in molecules useful for a range of biomedical applications. For example, such metalloproteins can serve as electron microscopy contrast agents [Ellisman et al. (2012), Picking faces out of a crowd: genetic labels for identification of proteins in correlated light and electron microscopy imaging. Methods in cell biology, 111: p. 139-155], probes for magnetic resonance imaging [Matsumoto and Jasanoff (2013), Metalloprotein-based MRI probes. FEBS letters, 587(8): p. 1021-1029], or as targeted radioactive tracers for radiotherapy and diagnostic imaging applications [Sawyer et al. (1992), Metal-binding chimeric antibodies expressed in Escherichia coli. Proceedings of the National Academy of Sciences, 89(20): p. 9754-9758].

However, the metal-binding proteins currently available in the state of the art are very often associated with disadvantages. Either they have a complex structure and can therefore only be produced with great effort. Some metal-binding proteins have a low, non-satisfactory affinity. Or they are characterized by thermal and proteolytic instability. Very often, known metal-binding proteins have low binding capacity, i.e., the number of bound metal ions per molecule mass is low, and they have a relatively high metal dissociation rate. It also happens that many of the known metal-binding proteins are unstable, poorly soluble or form oligomers in medium and in cells. All these disadvantages make the known prior art metal-binding proteins unsuitable for biomedical applications.

It is therefore an object of the present invention to provide a metal-binding polypeptide and/or protein that avoids or at least reduces at least some of the disadvantages of the metal-binding proteins described in the prior art.

SUMMARY OF THE INVENTION

The object underlying the invention is solved by the provision of a polypeptide comprising an amino acid sequence having at least 60% and at most approx. 98% homology with the amino acid sequence of copper storage protein from *Methylosinus trichosporium* OB3b (Csp1).

The object underlying the invention is also solved by a protein comprising:

a) a single polypeptide chain derived from copper storage protein from *Methylosinus trichosporium* OB3b (Csp1);

b) a bundle of four amphiphatic α-helices located on said single polypeptide chain;

c) three amino acid linkers that connect contiguous bundle-forming α-helices;

wherein the protein comprises at least one metal binding site.

According to the invention, the term "protein" as used herein, describes a macromolecule comprising one or more polypeptide chains. A "polypeptide" refers to a series of amino acid residues, connected one to the other typically by peptide bonds between the alpha-amino and carbonyl groups of the adjacent amino acids. The length of the polypeptide is not critical to the invention as long as the correct epitopes are maintained, e.g., metal-binding epitope(s). The term "polypeptide" is meant to refer to molecules containing more than about 30 amino acid residues.

The secondary structure is the three-dimensional form of local segments of proteins or polypeptide chains. The two most common secondary structural elements are α-helices and β-sheets, though β-turns and omega loops occur as well. Secondary structural elements typically spontaneously form as an intermediate before the protein or polypeptide chain folds into its three-dimensional tertiary structure.

The tertiary structure is the three-dimensional shape of a protein or polypeptide chain. The tertiary structure of a protein is the three-dimensional arrangement of multiple secondary structures belonging to a single polypeptide chain. Amino acid side chains may interact in different ways including hydrophobic interactions, salt bridges, hydrogen bonds, van der Waals forces and covalent bonds. The interactions and bonds of side chains within a particular protein or polypeptide chain determine its tertiary structure. The tertiary structure is defined by its atomic coordinates. A number of tertiary structures may fold into a quaternary structure.

The term "amino acid sequence" as used herein, refers to the sequence of amino acid residues of a protein. The amino acid sequence is usually reported in an N-to-C-terminal direction.

In the present invention, the term "homologous" refers to the degree of identity between sequences of two amino acid sequences, i.e., peptide or polypeptide sequences. The aforementioned "homology" is determined by comparing two sequences aligned under optimal conditions over the sequences to be compared. Such a sequence homology can be calculated by creating an alignment using, for example, the ClustalW algorithm. Commonly available sequence analysis software, more specifically, Vector NTI, GENETYX or other tools are provided by public databases.

"Percent homology" or "percent homologous" in turn, when referring to a sequence, means that a sequence is compared to a claimed or described sequence after alignment of the sequence to be compared (the "Compared Sequence") with the described or claimed sequence (the "Reference Sequence"). The percent homology (synonym: percent identity) is then determined according to the following formula: percent identity = 100 [1 -(C/R)]

wherein C is the number of differences between the Reference Sequence and the Compared Sequence over the length of alignment between the Reference Sequence and the Compared Sequence, wherein

(i) each base or amino acid in the Reference Sequence that does not have a corresponding aligned base or amino acid in the Compared Sequence and

(ii) each gap in the Reference Sequence and

(iii) each aligned base or amino acid in the Reference Sequence that is different from an aligned base or amino acid in the Compared Sequence, constitutes a difference and

(iv) the alignment has to start at position 1 of the aligned sequences;

and R is the number of bases or amino acids in the Reference Sequence over the length of the alignment with the Compared Sequence with any gap created in the Reference Sequence also being counted as a base or amino acid.

If an alignment exists between the Compared Sequence and the Reference Sequence for which the percent identity as calculated above is about equal to or greater than a specified minimum Percent Identity then the Compared Sequence has the specified minimum percent identity to the Reference Sequence even though alignments may exist in which the herein above calculated percent identity is less than the specified percent identity.

Methods for comparing the identity/homology of two or more sequences are known in the art. For example, the "needle" program, which uses the Needleman-Wunsch global alignment algorithm [Needleman and Wunsch (1970), J. Mol. Biol. 48:443-453] to find the optimum alignment (including gaps) of two sequences when considering their entire length may be used. The needle program is for example available on 30 the World Wide Web site and is further described in the following publication [EMBOSS: The European Molecular Biology Open Software Suite (2000) Rice, P. Longden, I. and Bleasby, A. Trends in Genetics 16, (6) pp. 276-277]. The percentage of identity between two polypeptides, in accordance with the disclosure, is calculated using the EMBOSS: needle (global) program with a "Gap Open" parameter equal to 10.0, a "Gap Extend" parameter equal 35 to 0.5, and a Blosum62 matrix.

An amino acid sequence which is "approx. at least 60% and at most approx. 98% homologous" refers to an amino acid sequence having, over its entire length, at least about 60%, or more, in particular about 61%, about 62%, about 63%, about 64%, about 65%, about 66%, about 67%, about 68%, about 69%, about 70%, about 71%, about 72%, about 73%, about 74%, about 75%, about 76%, about 775, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, and at most about 98% sequence identity with the entire length of a reference sequence, such as the amino acid sequence of copper storage protein from *Methylosinus trichosporium* OB3b (Csp1).

The protein according to the invention which is "derived from" Csp1 means a protein having a homology on the level of the amino acids of at approx. at least 60% with *Methylosinus trichosporium* OB3b (Csp1), as defined above.

The term "α-helix" as used herein, indicates a right-handed spiral conformation of a polypeptide chain or of a part of a polypeptide chain. In an α-helix, every backbone N-H group donates a hydrogen bond to the backbone C=0 group of the amino acid three or four residues earlier along the polypeptide chain.

A "bundle of four α-helices" as used herein, is defined as a protein fold composed of four α-helices that are nearly parallel or antiparallel to each other. An α-helix that contributes to the bundle of four α-helices is called a "bundle-forming α-helix". The four α-helices that form the bundle of four α-helices are located on a single polypeptide chain.

"Amphiphatic" means in this connection that the protein according to the invention possesses both hydrophobic and hydrophilic amino acids. The four-helix-bundle architecture [see Kamtekar and Hecht (1995), The four-helix bundle: what determines a fold?, FASEB Vo. 11, Issue 11, pp. 1013-1022] is characterized by hydrophobic inter-helical positions, and hydrophilic residues pointing to the exterior.

In the protein according to the invention an amino acid linker connects two α-helices that are located on the same polypeptide chain. The term "amino acid linker" as used herein, refers to a sequence of amino acids that is located between the C-terminal end of a first α-helix and the N-terminal end of a second α-helix, wherein the amino acids of the amino acid linkers are not part of any of the α-helices. Two α-helices are said to be contiguous because they are located on the same polypeptide chain and are directly connected by an amino acid linker. The length of an amino acid linker is defined as the number of amino acid residues that constitute the linker.

The term "binding site", as used herein, refers to one or more regions of the protein according to the invention that, as a result of its shape, favorably associate with another chemical entity or compound. A "metal binding site" as used herein, refers to one or more regions of the protein that favorably associate with a metal ion or atom. The shape of a protein-based binding site is determined by a set of amino acids with specific molecular interaction features and a defined spatial arrangement towards each other.

The skilled person is aware of methods to determine structural features of a protein such as α-helices or beta-sheets and/or linker sequences between such structures. The most common methods to determine the three-dimensional structure of a protein are X-ray crystallography, NMR spectroscopy and cryo-electron microscopy. These methods may be applied to detect the position and lengths of α-helices in a protein and the amino acids involved in the formation of these α-helices. Further, the methods may be applied to determine the length of amino acid linkers between two contiguous α-helices located on the same polypeptide chain and to identify the amino acids that form these linkers (i.e., the position and length of such linkers in the amino acid sequence), if these linkers are structured. In addition, these methods may be applied to determine the orientation of α-helices towards each other, for example parallel or antiparallel orientation, within a protein. Further biophysical methods that may be applied to determine secondary structures of proteins include circular dichroism (CD) spectroscopy and Fourier-transform infrared (FTIR) spectroscopy.

Alternatively, structural features of proteins such as, for example, the lengths of α-helices and/or amino acid linkers, may be predicted by using computational methods that start from the primary amino acid sequence of a protein. Several computer programs are known in the art that may be applied for the prediction of secondary protein structures. By way of non-limiting example, suitable computer programs include Psipred [McGuffin et al. (2000), The PSIPRED protein structure prediction server, Bioinformatics Vol. 16, Issue 4, pp. 404-405], SPIDER2 [Yang et al. (2016), SPIDER2: A package to predict secondary structure, accessible surface area, and main-chain torsional angles by deep neural networks], PSSPred [https://zhanglab.ccmb.med.umich.edu/PSSpred/], DeepCNF [Wang et al. (2016), Protein secondary structure prediction using deep convolutional neural fields, Scientific Reports 6, 18962]. One or more computer programs may be used for the prediction of a protein structure. Adaptation of the settings may be required to be able to directly compare the results of the different programs. The computer programs may be used in combination with experimental data to refine the results of the computational prediction.

The copper storage protein from *Methylosinus trichosporium* OB3b (Csp1) refers to a copper binding protein which has been described in the aerobic and methane-oxidizing bacterium *Methylosinus trichosporium* strain OB3b. The amino acid sequence of Csp1 can be retrieved from RCSB PDB entry no. 5FJD or Uniprot A0A0M3KL60. Said bacterium naturally uses Csp1 to store large quantities of copper for the membrane-bound particulate methane monooxygenase. Natural Csp1 is a tetramer of four-helix bundles with each monomer binding up to 13 Cu(I) ions.

The inventors were able to realize that, when considering using natural, wild-type Csp1 as a copper binding polypeptide in various applications, such as for metal decontamination or radio-imaging, it has several disadvantages: These include 1) its overall instability, 2) its oligomeric state (a tetrameric form), and 3) its low bacterial production yield and complex purification requirements.

The polypeptide and protein according to the invention, however, overcome these disadvantages. As the inventors were able to discover using computational protein design methods, a polypeptide that has an amino acid sequence in the specified homology range with respect to natural, wild-type Csp1 no longer has all these disadvantages. In contrast, it can bind metals in different oxidation states, such as Cu(II), Pb(II), and Co(II), and remains structured upon metal binding. The polypeptide and protein according to the invention binds transition ions with ultra-high affinities, has a low dissociation rate, high metal binding capacity, i.e., high metal:protein binding ratio. In particular, it has significantly increased thermal and proteolytic stability, is in monomeric form, and can be readily produced in standard expression systems at high yields. Furthermore, it is characterized by high solubility in aqueous media and in cells. Therefore, the polypeptide according to the invention is excellently suited for various biomedical applications.

Biomedical applications for which the polypeptide and protein of the invention is particularly suitable include, for example, the following: genetically-encodable radiotracers for radio-imaging for diagnostic applications, e.g., positron emission tomography scans; genetically-encodable protein tags for radio-tracing of specificity of protein-based therapeutics, e.g., monoclonal antibodies; as an emergency antidote for metal intoxication or disorders of metal metabolism that are characterized by excessive deposition of metals, e.g., copper, e.g. Wilson disease for copper metabolism, e.g., by oral or parenteral administration routes; genetically-encodable protein tag for targeted radioimmunotherapy; electron microscopy contrast agent for molecular and cellular labelling; industrial-scale precious metal-salvage; metal-decontamination and bioremediation of metal-contaminated environment; diverse *in vivo* animal experiments; etc.

It is self-evident to a person skilled in the art that all features, properties, advantages, etc., disclosed for the polypeptide according to the invention apply correspondingly to the protein according to the invention, without the need for explicit reference thereto.

The object underlying the invention is herewith fully achieved.

In an embodiment of the invention said polypeptide is configured to assemble into a monomeric metal binding protein, preferably a transition metal binding protein.

Surprisingly, the inventors found that the polypeptide according to the invention, although monomeric and not tetrameric like its natural counterpart, has a large number of metal-binding sites and possesses a particularly high binding affinity. The monomeric structure means a considerable facilitation in a recombinant preparation of the polypeptide.

"Transition metals" according to the invention include chemical elements with atomic numbers from 21 to 30, 39 to 48, 57 to 80, and 89 to 112, such as Cu(II), Pb(II), and Co(II).

In an embodiment of the invention said metal is, therefore, selected from Cu(II), Pb(II), and Co(II).

This measure has the advantage of providing such a polypeptide which is capable of binding metals that play an important role in the field of biomedical applications.

In a still further embodiment of the invention the amino acid sequence of the polypeptide comprises at most approx. 95%, preferably at most approx. 90%, further preferably at most approx. 85, and highly preferably at most approx. 81% homology with the amino acid sequence of Csp1.

The inventors have found that adjusting the amino acid sequence homology into the indicated range yields particularly suitable derivatives of Csp1, i.e., those that are particularly stable, monomeric, easy to purify, and have a very high affinity for metals.

In another embodiment of the polypeptide of the invention the amino acid sequence of Csp1 is SEQ ID NO: 1 (WT Csp1).

This measure advantageously provides a reference amino acid sequence that facilitates the skilled person to prepare Csp1 derivatives according to the invention in the desired homology range.

In an embodiment the protein according to the invention has a melting temperature ($T_m$) of at least ≥50°C, preferably of at least ≥100°C, and/or wherein it has an aggregation temperature ($T_{agg}$) of at least ≥50°C, preferably of at least ≥100°C.

This measure has the advantage of providing the protein of the invention in a form that gives sufficient stability to allow it to be processed, formulated and stored for extended periods of time. The protein melting point ($T_m$) is defined as the temperature at which the protein denatures. The aggregation temperature ($T_{agg}$) detects the onset of aggregation; the temperature at which molecules have a tendency to aggregate together. It can, for example, be determined by differential scanning calorimetry (DSC) or with circular dichroism (CD). The temperature at which a protein is fully denatured or aggregates depends on various factors, for example, the solvent and buffer conditions, a bound ligand, pressure and the temperature ramp rate that is applied to the protein. Within the present invention, the thermal stability of the protein of the invention was tested in a buffer comprising HEPE and NaCl, pH 7.4 and the temperature was increased at a rate of 1°C (Celsius) per minute. The melting temperature ($T_m$) may be extracted from a melting curve and corresponds to the temperature at which 50% of the protein is unfolded (see Embodiments, Material and Methods, 'Thermostability analysis', for an exemplary embodiment to define the $T_m$). Accordingly, the melting temperature is defined as the melting curve inflection mid-point.

In yet another embodiment the protein according to the invention binds metal with a dissociation constant ($K_D$) of at least ≤ 1fM, preferably of at least ≤ 1 fM.

This measure provides a protein with a particularly high affinity for metals. Binding affinity may be quantified by measuring an (equilibrium) dissociation constant ($K_D$), which refers to the dissociation rate constant ($k_D$, time$^{-1}$) divided by the association rate constant ($k_a$, time$^{-1}$ M$^{-1}$). $K_D$ can be determined by measurement of the kinetics of complex formation and dissociation, e.g., using Surface Plasmon Resonance (SPR) methods, e.g., a Biacore™ system (for example, using the method described in the Material-and-Methods section below); kinetic exclusion assays such as KinExA®; and BioLayer interferometry (e.g., using the ForteBio® Octet® platform). As used herein, "binding affinity" includes not only formal binding affinities, such as those reflecting 1:1 interactions between a polypeptide and its target, but also apparent affinities for which KJs are calculated that may reflect avid binding. The method described in the Material-and-Methods section is an example of obtaining the $K_D$ through competitive binding assay with a chromophoric probe with a known $K_D$ to Cu(II) ion.

In an embodiment of the protein according to the invention each amino acid linker has a length of between 2 and 20, preferably between 2 and 15, more preferably between 2 and 10, and most preferably between 3 and 7 amino acids.

The inventors were able to find out that such lengths of the linkers result in optimum binding activity. Without being bound to theory, the shorter linkers may presumably contribute to the improved stability of these protein.

In another embodiment of the protein according to the invention each $\alpha$-helix comprises one or more cysteine residue(s). Where the frequency of cysteine residues along an $\alpha$-helix sequence is at least one cysteine is located at every third position $(XXC)_n$, and at most one cysteine at every 11th position $(XXXXXXXXXXC)_n$. The $\alpha$-helices presented in this invention cover such helical occurrence frequency range.

In yet another embodiment of the invention the polypeptide comprises the following amino acid sequence SEQ ID NO: 2:

$$X_1X_2GAX_3YX_4ALLX_5SSX_6X_7CVX_8VGEX_9X_{10}LX_{11}HX_{12}X_{13}EMLX_{14}X_{15}NDX_{16}SMGAX_{17}TKAX_{18}X_{19}DLVX_{20}ACX_{21}X_{22}LAKLAGTX_{23}SAX_{24}TPX_{25}X_{26}AX_{27}X_{28}VAX_{29}VCX_{30}X_{31}CX_{32}KEX_{33}DKX_{34}PSX_{35}X_{36}EX_{37}KX_{38}X_{39}X_{40}EACX_{41}X_{42}CAEECX_{43}KVA,$$

wherein:

$X_1$ = M or missing,
$X_2$ = M or H,
$X_3$ = KorH,
$X_4$ = K, A or E,
$X_5$ = D, E or R,
$X_6$ = S, R or E,
$X_7$ = H or R,
$X_8$ = A, R or K,
$X_9$ = D, R or E,
$X_{10}$ = C, AorW,
$X_{11}$ = R or E,
$X_{12}$ = C or A,
$X_{13}$ = F, R or Q,
$X_{14}$ = A, R, K or E,
$X_{15}$ = M, R or K,
$X_{16}$ = A or E,
$X_{17}$ = C, LorA,
$X_{18}$ = T, F or A,
$X_{19}$ = Y or E,
$X_{20}$ = A or K,
$X_{21}$ = G, A or E,
$X_{22}$ = A, E or R,
$X_{23}$ = N or E,
$X_{24}$ = F, R or Q,
$X_{25}$ = A, K, R or E,
$X_{26}$ = F, K, R or L,
$X_{27}$ = K or A,
$X_{28}$ = V, Q, R or E,
$X_{29}$ = D or R,
$X_{30}$ = A, E or R,

X_31 = A, K, R or Q,
X_32 = K, Q or A,
X_33 = C or A,
X_34 = F orW,
X_35= I, M orY,
X_36 = A or E,
X_37 = C or A,
X_38 = A or E,
X_39 = C or A,
X_40 = G or A,
X_41 = Q, K or E,
X_42 = A, K, R or E,
X_43 = H, R, K or E.

The inventors were able to identify a consensus amino acid sequence derived from wild-type Csp1 that yields a polypeptide that has the desired properties. This measure has the advantage that a person skilled in the art has a range of variation options. That is, at positions $X_1$-$X_{43}$, the respective amino acids indicated can be used as desired. It is understood that according to the invention, all arbitrary and conceivable combinations of $X_1$_$X_{43}$ as indicated are encompassed and disclosed.

In another embodiment the polypeptide according to the invention comprises the following amino acid sequence SEQ ID NO: 3:

MGAKYKALLESSRRCVRVGERCLRHCREMLRRNDASMGACTK
ATYDLVKACAELAKLAGTNSARTPKKAKQVARVCEKCKKECDKF
PSIAECKACAEACKKCAEECRKVA (plr1),

or the following amino acid sequence SEQ ID NO: 4:

MGAKYKALLRSSRRCVKVGEECLRHCREMLKRNDASMGACTK
ATYDLVKACARLAKLAGTNSARTPRRAKRVARVCERCKKECDK
FPSIAECKACAEACQRCAEECKKVA (plr2),

or the following amino acid sequence SEQ ID NO: 5:

MHGAKYKALLESSRRCVRVGERCLRHAREMLRRNDASMGALT
KATYDLVKACAELAKLAGTNSARTPKKAKQVARVCEKCKKECD
KWPSMAEAKACAEACKKCAEECRKVA (plr1_cr3),

or the following amino acid sequence SEQ ID NO: 6:

MHGAKYKALLESSRRCVRVGERALRHAREMLRRNDASMGAAT
KAFYDLVKACAELAKLAGTNSARTPKKAKQVARVCEKCKKEADK
WPSYAEAKAAAEACKKCAEECRKVA (plr1_cr61),

or the following amino acid sequence SEQ ID NO: 7:

MHGAKYKALLESSRRCVRVGERWLRHAREMLRRNDASMGAAT

KAAYDLVKACAELAKLAGTNSARTPKKAKQVARVCEKCKKEAD

KWPSMAEAKAAAEACKKCAEECRKVA (plr1_cr62),

or the following amino acid sequence SEQ ID NO: 8:

MHGAHYAALLESSERCVEVGERCLEHCQEMLEKNDESMGACT

KATEDLVKACEELAKLAGTESAQTPELAAEVARVCEQCQKECD

KFPSIEECKECAEACQECAEECEKVA (plr_neg1),

or the following amino acid sequence SEQ ID NO: 9:

MHGAHYEALLESSERCVEVGERCLEHCQEMLEKNDESMGACT

KATEDLVKACEELAKLAGTESAQTPELAAEVARVCRQCAKECDK

FPSIEECKECAEACEECAEECRKVA (plr1_neg2).

This measure provides specific Csp1 derivatives, designated by the inventors as 'plr1', 'plr2', 'plr1_cr3', 'plr1_cr61', 'plr1_cr62', 'plr_neg1', and 'plr1_neg2', which are particularly suitable according to their findings in accordance with the invention.

In a particularly preferred embodiment of the invention the polypeptide consists or consists essentially of an amino acid sequence according to SEQ ID NO: 2 to SEQ ID NO: 9.

"Consisting essentially of" shall mean that a polypeptide according to the pre-sent invention, in addition to the sequence according to any of SEQ ID NO: 2 to SEQ ID NO: 9 contains additional N- and/or C-terminally located stretches of amino acids that are not necessarily forming part of the polypeptide that functions as a metal binding epitope.

The polypeptides and/or proteins and/or nucleic acid molecules disclosed in accordance with the present invention may also be in "purified" form. The term "purified" does not require absolute purity; rather, it is intended as a relative definition, and can include preparations that are highly purified or preparations that are only partially purified, as those terms are understood by those of skill in the relevant art.

The polypeptides, proteins and nucleic acids according to the invention may be in "enriched form". As used herein, the term "enriched" means that the concentration of the material is at least about 2, 5, 10, 100, or 1000 times its natural concentration (for example), advantageously 0.01 %, by weight, preferably at least about 0.1% by weight. Enriched preparations of about 0.5%, 1%, 5%, 10%, and 20% by weight are also contemplated. The sequences, constructs, vectors, clones, and other materials comprising the present invention can advantageously be in enriched or isolated form.

Another subject-matter of the invention relates to a nucleic acid molecule encoding for the polypeptide and/or protein according to the invention, optionally linked to a promoter sequence.

As used herein the term "nucleic acid molecule" or, synonymously "polynucleotide" coding for (or encoding) a polypeptide/protein refers to a nucleotide sequence coding for the protein/polypeptide including artificial (man-made) start and stop codons compatible for the biological system the sequence is to be expressed. The term "promoter" means a region of DNA involved in the binding of the RNA polymerase to initiate transcription.

The polynucleotide or nucleic acid molecule coding for said polypeptide or protein may be synthetically constructed or may be naturally occurring. The nucleic acid or polynucleotide may be, for example, DNA, cDNA, PNA, RNA or combinations thereof, either single- and/or double- stranded, or native or stabilized forms of polynucleotides, such as, for example, polynucleotides with a phosphorothioate backbone, and it may or may not contain introns so long as it codes for the polypeptide. Of course, only peptides that contain naturally occurring amino acid residues joined by naturally occurring peptide bonds are encodable by a polynucleotide.

A still further aspect of the invention provides a vector, such as an ex-pression vector, capable of expressing the polypeptide and/or protein according to the invention.

The features, characteristics, advantages and embodiments disclosed for the polypeptide and protein according to the invention apply to the nucleic acid molecule and (expression) vector correspondingly.

Another subject-matter of the invention relates to a recombinant host cell comprising the polypeptide according to the invention, or the nucleic acid or the expression vector according to the invention, wherein said host cell preferably is selected from a bacterial (e.g., *E. coli),* yeast, insect, mammalian or human cell.

The features, characteristics, advantages and embodiments disclosed for the polypeptide and protein according to the invention apply to the host cell correspondingly.

Another subject-matter according to the invention relates to a pharmaceutical composition comprising at least one active ingredient selected from the group consisting of the polypeptide according to the invention, the protein according to the invention, the nucleic acid or the expression vector according to the invention, and the recombinant host cell according to the invention, optionally a pharmaceutically acceptable carrier, and further optionally, pharmaceutically acceptable excipients and/or stabilizers.

Said pharmaceutical composition is selected from the group consisting of: radio immunotherapeutic agent, radio tracing agent, contrast agent, antidot for metal intoxication, metal-decontamination agent, and metal recovery agent.

The features, characteristics, advantages and embodiments disclosed for the polypeptide and protein recited at the outset according to the invention apply to said pharmaceutical composition correspondingly.

A still further subject-matter according to the invention relates to a kit comprising:

(a) a container comprising the polypeptide, or the protein, or the nucleic acid molecule, or the expression vector, or the recombinant host cell, or the pharmaceutical composition according to the invention, in solution or in lyophilized formulation;

(b) optionally, a second container containing a diluent or reconstituting solution for the lyophilized formulation;

(c) optionally, instructions for (i) use of the solution or (ii) reconstitution and/or use of the lyophilized formulation.

The kit may further comprise one or more of (iii) a buffer, (iv) a diluent, (v) a filter, (vi) a needle, or (v) a syringe. The container is preferably a bottle, a vial, a syringe or test tube; and it may be a multi-use container. The pharmaceutical composition is preferably lyophilized.

The kit of the present invention preferably comprises a lyophilized formulation of the present invention in a suitable container and instructions for its reconstitution and/or use. Suitable containers include, for example, bottles, vials (e.g., dual chamber vials), syringes (such as dual chamber syringes) and test tubes. The container may be formed from a variety of materials such as glass or plastic. Preferably the kit and/or container contain/s instructions on or associated with the container that indicates directions for reconstitution and/or use. For example, the label may indicate that the lyophilized formulation is to be reconstituted to peptide concentrations as described above. The label may further indicate that the formulation is useful or intended for subcutaneous administration.

A still further subject-matter of the invention is the use of the polypeptide according to the invention for reconstituting a monomeric metal binding protein, preferably a transition metal binding protein.

Yet another subject-matter of the invention is a copper storage protein from *Methylosinus trichosporium* OB3b (Csp1) and/or a polypeptide comprising the amino acid sequence of SEQ ID NO: 1 for use as a medicament, preferably selected from the group consisting of: radio immunotherapeutic agent, radio tracing agent, contrast agent, antidot for metal intoxication, metal-decontamination agent, and metal recovery agent.

The inventors were able to realize that even the natural, wild-type copper storage protein from *Methylosinus trichosporium* OB3b (Csp1) can be used as a medicament, not only the polypeptides or protein according to the invention which are derived therefrom.

The features, characteristics, advantages and embodiments disclosed for the polypeptide or protein recited at the outset according to the invention apply to said uses correspondingly.

The invention is now further explained by means of embodiments resulting in additional features, characteristics and advantages of the invention. The embodiments are of pure illustrative nature and do not limit the scope or range of the invention. The features mentioned in the specific embodiments are features of the invention and may be seen as general features which are not applicable in the specific embodiment but also in an isolated manner in the context of any embodiment of the invention.

The invention is now further described and explained in further detail by referring to the following non-limiting examples and figures:

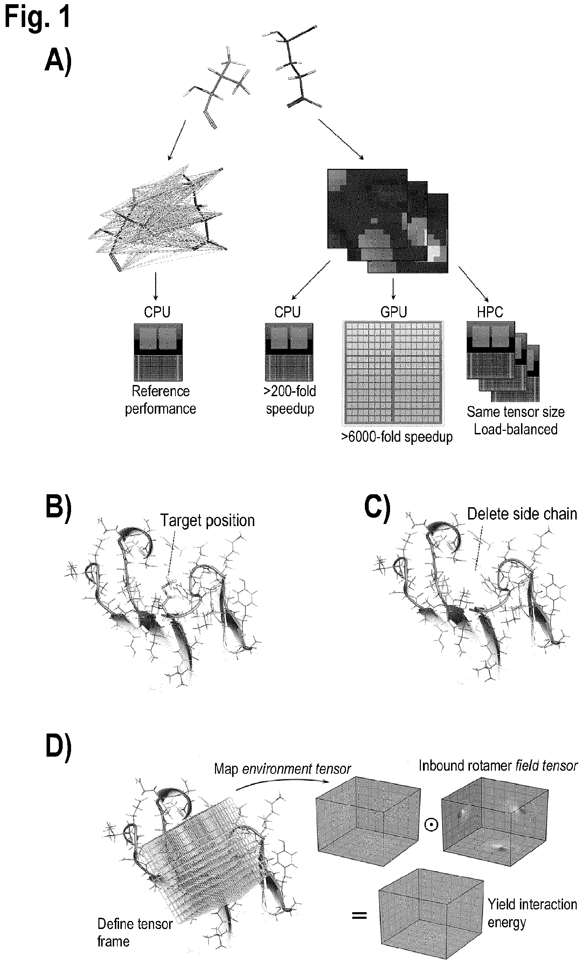

Fig. 1:     The acceleration concept of the Damietta design framework. (A) The non-bonded interactions between an amino acid and its molecular environment (e.g. another proximal amino acid) entail the calculation of all atom-atom pairwise potential energies within a distance cutoff. This necessitates programmatic loops to evaluate pairwise distances and energy equations that represents instructions, as well as intensive memory address handling that slows the overall performance. Thus, formulating the energy evaluation problem between two groups of atoms as a single tensor operation not only speeds up the scoring on conventional processors, but also renders the calculation highly compatible with stream processors. Moreover, the constant dimensions of the used tensors, enables ideal load balancing on high performance computers. The performance enhancement figures were calculated for the Lennard-Jones potential function, a cubic tensor representation of 22 Å side and $0.5\times0.5\times0.5$ Å voxels. The computing operations follow a scheme whereby for an input structure: (B) once a mutable or repackable target residue is defined, the structure is transformed to the frame of reference with respect the residue's backbone coordinates. (C) Then the side chain atoms of the target residue are deleted, leaving behind the "*environment*" atoms. (D) Proxy values for the atoms' positions, partial charges (illustrated here as plane projections; yellow: negative; blue: positive), or their surface solvation energies, are projected onto the voxels of a constructed tensor. The rotamer library comprise the more expensive, pre-computed smooth interaction fields (i.e., *field tensors;* example shown for an Asparagine side chain), which through a single element-wise multiplication with the *environment tensor* yields the spatially resolved energy in a "two-body" format.

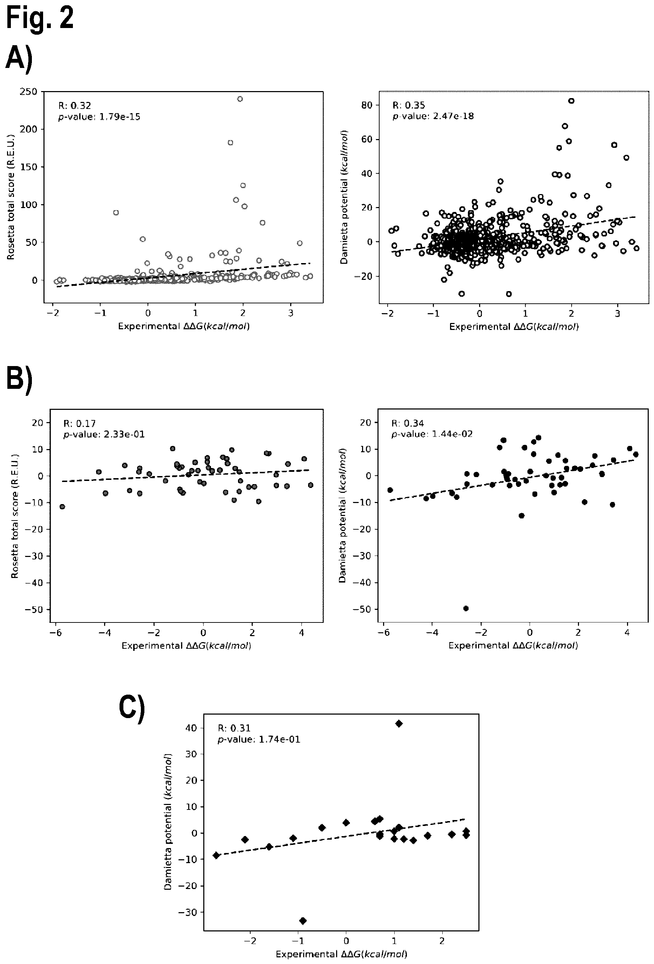

Fig. 2: Retrospective validation results of the Damietta potential against three different benchmarks. (A) A comparison between the Rosetta (left; brown) and Damietta (right; cyan) scores correlation with the change in folding free energy ($\Delta\Delta G$) values for mutants of G$\beta$1 protein. (B) A comparison between the Rosetta (left; brown) and Damietta (right; cyan) scores correlation with the experimental change in folding free energy ($\Delta\Delta G$) values for mutants of $\beta$-glucosidase. The $\Delta\Delta G$ values and the Rosetta scores were both reported in the respective studies (C) Evaluation of the Damietta energy function performance against a benchmark of mutants of solvent-exposed and charged residues from T4 lysozyme, human lysozyme, ribonuclease Sa and *B. subtilis* CSP-B proteins. The Pearson correlation coefficients and the correlation p-values are shown as insets in the respective plots.

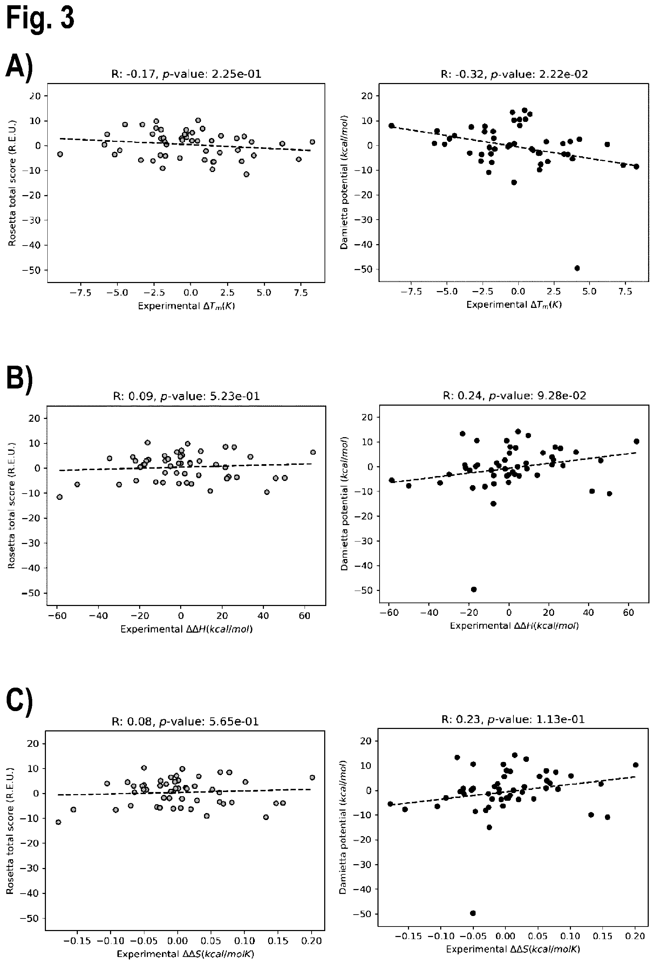

Fig. 3: Energy scoring correlations with other thermodynamic parameters from the $\beta$-glucosidase mutant dataset. Comparisons of the Rosetta and Damietta energy scores correlation with the $\beta$-glucosidase mutants' (A) change in melting temperature ($\Delta T\_m$), (B) change in folding enthalpy ($\Delta\Delta H$), and (C) change in folding entropy ($\Delta\Delta S$) indicate better correlations with the Damietta energy values.

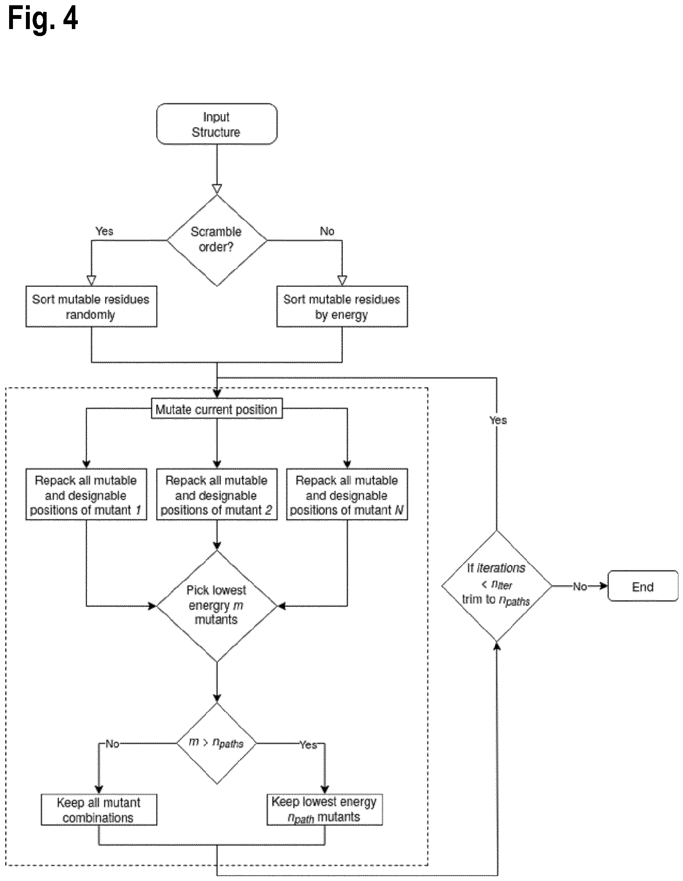

Fig. 4: A flow chart of the greedy branch-and-rank combinatorial sampling algorithm used for design. Scheme of the few-to-many-to-few combinatorial sampler. The algorithm assumes all mutable and repackable residues are inter-dependent, and follows a maximum number of paths $n_{paths}$ down the decision tree. At the start of the design simulation all of the combinatorially generated sequences are further designs so long as their number is $\leq n_{paths}$. But as the sequence combinations grow $> n_{paths}$, the algorithm ranks all of the designs by the average energy per residue (evaluated at the mutable and repackable positions). Only a small number (= $n_{paths}$) of lowest energy mutants kept for the next position mutagenesis round. The order of mutable positions can be preset or randomized as the defined by the user. The main flow of sampling therefore follows: i) mutate position, ii) calculate average energy of every mutant at that position, iii) combine the top m mutations with all of the previously kept mutants from the previous cycle to the evaluate the average energy per residue, iv) if the generated combinations are larger in number than $n_{paths}$, only keep the lowest energy $n_{paths}$ sequences, v) move to the next mutable position and repeat step (i) repeat these iterations over all mutable residues. This cycle is repeated $n_{iters}$ times for better convergence.

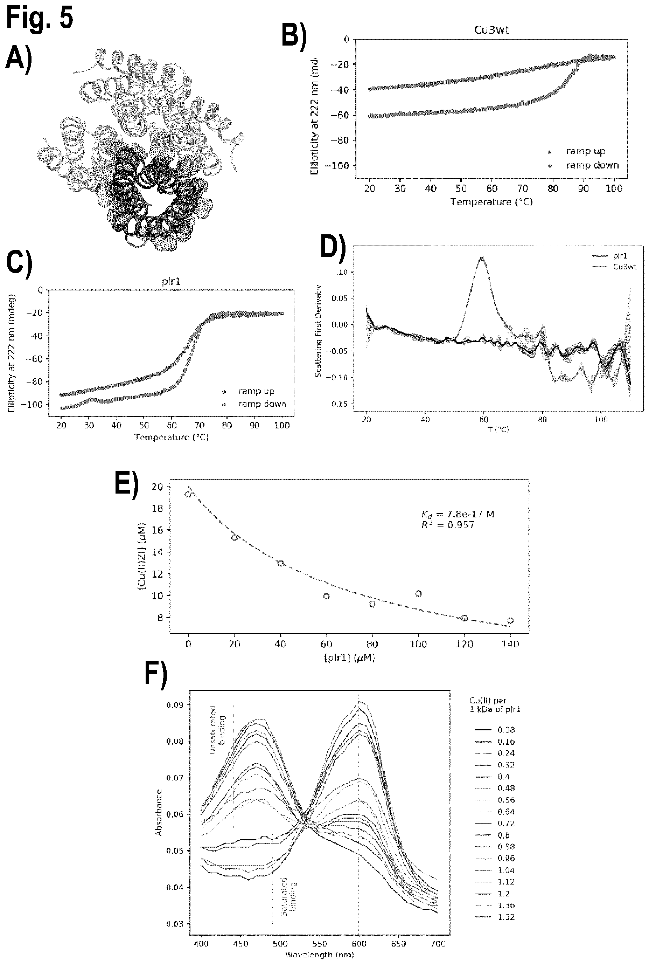

Fig. 5 Design of monomeric Cu(II)-binding protein. (A) Crystal structure of the tetrameric apo-Csp1 (PDB: 5FJD). Monomeric design template shown in red cartoon where the surface residues (blue surface) were designed to polar amino acids in the first design step. (B, C) CD melting curves of WT Csp1 shows irreversible unfolding (B), whereas the plr1 design has stronger helical character and unfolds and refolds reversibly (C). (D) NanoDSF melting curves show the onset of the WT Csp1 protein aggregation (as tracked by UV scattering) to be around 60 °C, i.e. before its $T_m$, which the CD shows to take place at 87 °C. In contrast, the plr1 design does not show any clear scattering inflection, and did not precipitate in the capillaries. (E) Competitive Cu(II) binding assay using zincon (ZI) as a high-affinity probe, shows a fit $K_d$ value of 78 aM for the plr1. (F) Evaluating the number of plr1 binding sites using the chromophoric change of ZI indicates at a mid-point between 12 and 13 Cu(II) binding sites, or approximately 1 metal binding site per 1 kDa of protein.

EMBODIMENTS

1. Material and methods

*Design workflow*

All the design processes described here were performed using an early version of the Damietta software, the main components of which are described herein. Given the large number of mutations introduced at each design stage, a combinatorial design algorithm was used, which deploys a few-to-many-to-few sampler (damietta_cs_f2m2f). The resulting decoys were further filtered according to their stability in accelerated molecular dynamics (AMD) simulations that follow a serial tempering routine previously described by Skokowa et al. (2022) Nat Commun 13, 2948. The two most conformationally homogeneous (quantified as the average all-vs.-all RMSD averaged across all frames output from the AMD simulations) designs (plr1 and plr2) in AMD simulations were accordingly selected for experimental characterization. The following sections describe the methods used for building a discrete backbone-dependent rotamer library, the Damietta energy function, and retrospective benchmarking.

*The Damietta rotamer library.*

Large-scale molecular dynamics simulations of capped amino acids (i.e., Ac-X-NHMe, where X represents the amino acid symbol) were used as pools from which representative rotamers were sampled. These trajectories can be performed under a predefined force field in explicit water, yielding a pool of freely inter-changing conformations. In the current implementation, the inventors have used a set of trajectories performed under the CHARMM27 force field in explicit solvent by. The 18 proteinogenic A, C, D, E, F, H, I, K, L, M, N, Q, R, S, T, V, W, Y, excluding G and P from the current implementation. The trajectories of every Ac-X-NHMe amino acid was 1-ps-long and was partitioned into 36 $\varphi,\psi$-bins (each with (t60, t60) intervals). The propensity of each backbone conformational state can represent its relative energy with respect to all of the other conformational states through a Boltzmann term:

$$\Delta G_{pp} = -k_b T \ln \frac{O_{(\varphi,\psi)_m}}{N}$$

Where $\kappa_b$ is the Boltzmann constant in kcal·mol$^{-1}$ K$^{-1}$, $T$ is the temperature in Kelvins (here taken as constant values of 0.001985875 and 298, respectively), $O_{(\varphi,\psi)m}$ is the number of observations in the $m^{th}$ ($_{\varphi,\psi}$) conformation bin, and $N$ is the total number of conformations. A similar explicit internal energy term is used to describe the side chain conformation preferences, where all conformations within each ($\varphi_i,\psi_j$) bin undergo k-means clustering, resulting in $k$ representative side chain conformers. Here the value of $k$ was set to 50 conformational clusters, and the representative conformer of each cluster was taken to be the one with the lowest RMSD to the average structure of the entire cluster, where the energy of every cluster is defined as:

$$\Delta G_k = -k_b T \ln \frac{O_{(k_n|(\varphi,\psi)_m)}}{O_{(\varphi,\psi)_m}}$$

Where $O_{(k_n}|(\varphi,\psi)_m)$ is the number of observations in conformation bin $(\varphi,\psi)_m$ of the $n^{th}$ cluster out of $k$ clusters. In cases where the entire molecular dynamics simulation results in a conformational bin that is underpopulated, i.e., $O_{(\varphi,\psi)m} < k$, the entire bin is not represented in the library.

*The Damietta energy function.*

The energy function is composed of 5 terms representing the energy different to a ground state of a solvated, capped amino acid, as follows: backbone internal energy ($\Delta G_{pp}$), side chain conformational energy ($\Delta G_{k}$), Lennard-Jones interaction energy ($\Delta G_{LJ}$), solvation energy ($\Delta G_{solv}$), and electrostatic interaction energy ($\Delta G_{elec}$). The total energy is a weighted sum of these terms, as:

$$\Delta G_{total} = w_{pp}\Delta G_{pp} + w_k\Delta G_k + w_{LJ}\Delta G_{LJ} + w_{solv}\Delta G_{solv} + w_{elec}\Delta G_{elec}$$

The energy calculation scheme follows a two-body formulation whereby the interactions are only calculated between two sets of atoms belonging to the *1st-body* and the *2nd-body*. The *1st-body* atoms are the all the atoms within a bounding box of dimensions $d_{box} \times d_{box} \times d_{box}$ excluding the side chain atoms of the mutable residue, where the bounding box is centered at the C$\alpha$ atom of the mutable residue. The *2nd-body* atoms represent the side chains of the rotamer to be placed in the mutable residue position, as sampled from the rotamer library. This scheme applies to the interaction energy terms (e.g., $\Delta G_{LJ}$ and $\Delta G_{elec}$) as well as the solvation free energy term ($\Delta G_{solv}$). To maximise the compatibility between different energy terms, the partial charges and Lennard-Jones parameters were obtained from the CHARMM27 force field parameters, and the surface-area based solvation energy term relied on the CHARMM19-based parameters for the EEF1 model. Moreover, the $\Delta G_{pp}$ and $\Delta G_k$ terms, described above, are derived from conformational distributions extracted from simulations that used the CHARMM27 force field.

For evaluating electrostatic interaction energies, the following form of the Generalised Born model was used. The interactions are calculated between the *1st-body* atoms $i \in I$ of the protein where the mutable residue side chain atoms are deleted, and $I$ protein atoms exist within a bounding simulation box from one side, and the *2nd-body* atoms $j \in J$ that constitute the inbound side chain atoms looked up from the rotamer library. The original form of the function was:

$$\Delta G_{elec} = \frac{322 q_i q_j}{\varepsilon(r) r_{ij}} - \frac{322 q_i q_j}{\left( r_{ij}^2 + b_i b_j e^{-\frac{r_{ij}^2}{4 b_i b_j}} \right)^{\frac{1}{2}}} \left( \frac{1}{\varepsilon_p} - \frac{1}{\varepsilon_s} \right) - 166 \frac{q_j^2}{b_j} \left( \frac{1}{\varepsilon_p} - \frac{1}{\varepsilon_s} \right)$$

Where $q_i$ and $q_j$ represent the partial charges of atoms i and j, separated by the distance $r_{ij}$. A distance-dependent dielectric function of $\varepsilon(r) = r$ was assumed, since the electrostatic interactions cutoff was set to 7.0 Å, the value of $\varepsilon(r)$ ranged as $\sigma_{LJ} < \varepsilon(r) < 7.0$, where $\sigma_{LJ}$ of a carbon-carbon interaction, for example, is approximately 4 Å. $\varepsilon_p$ and $\varepsilon_s$ represent the dielectric constant of protein core ($\varepsilon_p = 4$) and water ($\varepsilon_s = 80$), respectively. The born radii $b_i, b_j$ represent the closest distance of the respective atoms to the solvent.

The above function was altered to lower its computing cost, whereby the first and second and terms are factored after dividing the second term by $\varepsilon(r) r_{ij}$. Also, in the second term, the average interatomic distance in the simulation cube

$\bar{r}_{ij}^2$ was used instead of the individual distances, and born radius was taken as the average born radius in the simulation cube; $b_i = b_j = \bar{b}$. The last charge solvation term was offset by a factor of $\frac{1}{0.13 d_{box}}$ to obtain a positive energy penalty for burying a charge at a born radius of $0.13 d_{box}$, where $d_{box}$ is the length of one dimension of the simulation box. This yielded the following form:

$$\Delta G_{elec} = \frac{322 q_i q_j}{\varepsilon(r) r_{ij}} \left( 1 - \frac{1}{\left( \bar{r}_{ij}^2 + \bar{b}^2 e^{-\frac{\bar{r}_{ij}^2}{4 \bar{b}^2}} \right)^{\frac{1}{2}}} \left( \frac{1}{\varepsilon_p} - \frac{1}{\varepsilon_s} \right) \right) - 166 \frac{q_i^2}{\bar{b}} \left( \frac{1}{\varepsilon_p} - \frac{1}{\varepsilon_s} \right) \left( 1 - \frac{1}{0.13 d_{box}} \right)$$

The Lennard-Jones function was implemented as a piece-wise function to avoid extreme sensitivity to interatomic clashes; as such clashes are mostly expected to be relaxed upon MD-based minimization. The piece-wise function consists of three components; a standard LJ term in the attractive range of inter-atomic distances, a slow-growing repulsive term across a band of the atomic crust, and a flat maximum at a defined atomic core, as follows:

$$\Delta G_{LJ} = \begin{cases} \varepsilon_{LJ,j}\left(\dfrac{\sigma_{LJ,i}^{12}}{r_{ij}^{12}} - \dfrac{\sigma_{LJ,i}^6}{r_{ij}^6}\right) & (\sigma_{LJ,j} - c_{soft}) < r_{ij} \leq c_{lr} \\[2em] \varepsilon_{LJ,j}\dfrac{\sigma_{LJ,i}^2}{r_{ij}^2} & (\sigma_{LJ,j} - c_{hard}) < r_{ij} \leq (\sigma_{LJ,j} - c_{soft}) \\[2em] \varepsilon_{LJ,j}\dfrac{\sigma_{LJ,j}^{12}}{\sigma_{LJ,j} - c_{hard}} & 0 < r_{ij} \leq (\sigma_{LJ,j} - c_{hard}) \end{cases}$$

Where $\varepsilon_{LJ,j}$ and $\sigma_{LJ,j}$ are the minimum LJ energy value (in kcal·mol$^{-1}$) and the LJ radius (in Å) of atom $j$ as obtained from the CHARMM27 parameters. The use of $\varepsilon_{LJ,j}$ and $\sigma_{LJ,j}$ parameters of atom j instead of the averaged parameters for atoms $i$ and $j$ was aimed at lowering the computing cost, since the entire LJ interaction fields are pre-calculated for inbound rotamer atoms (i.e., $j \in J$ atoms). Both the LJ and electrostatic interaction fields are calculated for all values of $0 < r_{ij} \leq c_{lr}$ at a resolution of 0.5 Å, where $c_{lr}$ is the long-range cutoff that is set to 7.0 Å. Such fields are stored in the chemical library provided with the software, and are looked up during the design process depending the mutable position ($\varphi, \psi$) bin.

A generic term solvation free energy term based on a surface area method was also put in place to account for the hydrophobic effect. This term was adapted from the EEF1 energy model parameters previously described. The solvation term follows the general form:

$$\Delta G_{solv} = \sum_l \sigma_{solv,l} A_l(\boldsymbol{r})$$

Where $\sigma_{solv,l}$ is the solvation energy per unit surface area (in kcal·mol$^{-1}$Å$^{-2}$) of atom $l$, with solvent-exposed surface area $A_l$ when located at position vector $\boldsymbol{r}$. A rough approximation of function Al is the non-occluded vdW surface area of slightly inflated vdW radii (this inflation was performed here by an added 0.5 Å to the atomic vdW radii). This energy term is calculated separately for the $1^{st}$-body atoms ($\Delta G_{solv,\,1}$), the $2^{nd}$-body ($\Delta G_{solv,2}$), and the $1^{st}$-body and $2^{nd}$-body combined ($\Delta G_{solv,1,2}$). These values would represent the solvated free energies of the protein environment with the removed side chain atoms at the mutable residue, the inbound side chain atoms of a rotamer from the rotamer library, and the combined protein environment and rotamer side chain atoms after rotamer placement, respectively. Given these three values, the final solvation free energy term is calculated as follows:

$$\Delta G_{solv} = \Delta G_{solv,\,1,2} - (\Delta G_{solv,\,1} + \Delta G_{solv,\,2})$$

It is worth noting that the benchmarking and design data represented here is based on the EEF1 solvation potential (using Damietta versions up to 0.23). From v0.32 onwards, the inventors have implemented an extended version of the EEF1-SB solvation potential, featuring accuracy improvement, but is not discussed within the scope of this study. While the different terms of the energy function are compatible as they were derived from the same force field, the softening of the repulsive component of the LJ term necessitates the downscaling of the electrostatic term, in order to avoid highly clashy configurations with optimal electrostatic interactions. Additionally, the $w_k$ is recommended to be set to 0 for mutagenesis tasks, and to 1 for repacking tasks. This is as $\Delta G_k$ is not directly comparable across different amino acid types, given that different amino acid types have drastically different chemical exchange timeframes in solution, even of their backbone is fixed. Otherwise, the other weighting factors were all set to 1.0; $w_{pp} = w_{LJ} = w_{solv} = 1.0$. Additionally, to deploy some tiered scoring to avoid calculating all energy terms for highly clashy rotamers, a maximum LJ energy value was set to 30 kcal·mol$^{-1}$ to disqualify clashy rotamers from undergoing other energy term evaluations.

*Evaluation of scoring accuracy against different benchmarks*

Ability of Damietta framework to evaluate stability of protein mutants was benchmarked using three independent experimental datasets of: 1) mutants of the β1 domain of streptococcal protein G (PDB ID: 1PGA); 2) mutants of β-glucosidase B from *P. polymyxa* (PDB: 2JIE); 3) charge reversal or charge neutralization mutants of T4 lysozyme (PDB: 3LZM), human lysozyme (PDB: 1REX), ribonuclease Sa (PDB: 1C54), and *B. subtilis* cold shock protein B (PDB: 1CSP). Generating mutants and estimating their energies was done using single-point (sp) Damietta routine with the parameters

(-max_lj 100.0, -w_pp 1.0, -w_k 0.0, -w_lj 1.0, -w_solv 1.0, -w_elec 0.125). $\Delta\Delta G$ was calculated as the difference in free energy between the mutant and the wild type reference. Predictive potential of a software was assessed using a Pearson correlation coefficient (R) for computed energy values against experimental data. Performance of Damietta was analyzed in comparison with performance of Rosetta framework described before.

*Design and characterization of copperbinders*

Metal-binding proteins serve the essential functions including catalysis, sensing, transport, and storage. Designed metalloproteins can be tailored to encode one or more of such functions. Particularly, the combined design objective of engineering proteins capable of high-affinity metal binding, efficient storage, and transport, can result in molecules useful for a range of biomedical applications. For example, such metalloproteins can serve as electron microscopy contrast agents, probes for magnetic resonance imaging, or as targeted radioactive tracers for radiotherapy and diagnostic imaging purposes. Recently, a cysteine-rich helical bundle protein (Csp1) was discovered in methanotrophs. This natural copper-binding template was shown to bind Cu(I) at high stoichiometry (13:1 Cu(I):protein ratio), to have a low molecular weight (<13 kDa), and a simple helical structure. The several drawbacks however associated with Csp1 are: 1) its overall instability, 2) its oligomeric state (a tetrameric form), and 3) its low bacterial production yield and complex purification requirements. The inventors thus sought out to computationally redesign the protein sequence to overcome these three challenges.

Combinatorial design simulations using the Damietta software were run to redesign the template structure of apo-protein form (PDB: 5FJD). 22 designable residues and 13 repackable residues were defined for combinatorial mutational and conformational optimization. Mutational targets were mostly aimed at surface residues with the tasks of: disrupting the oligomerization interface of the tetrameric Csp1 template, improving the protein solubility, and increasing the bundle's structural stability. A hundred design simulation replicas were spawned with a randomized order of the mutational decision tree, where each mutable position represents a node, using the few-to-many-to-few (f2m2f) combinatorial sampling algorithm. The resulting unique-sequence decoys were further filtered by molecular dynamics (MD) simulations to evaluate their conformational stability (as previously described). The two most conformationally stable designs (named plr1 and plr2) were selected for experimental characterization (Table 1). Another round of computational design was also performed to optimize non- exposed residues, these decoys were not experimentally tested as MD simulations indicated significant structural instability compared to the polar designs. In a second design step, we sought to create constructs with a more sealed core. This was done by mutating 3 or 6 cysteine residues (and their surrounding positions) into well-packed hydrophobic residues at the end of the cysteine-lined lumen of the plr1 model. Three such design candidates were also synthesized and tested, plr1_cr3, plr1_c61, and plr1_cr62. Moreover, designs based on plr1 were created to be negatively supercharged by biasing the redesign of the polar residues towards negatively charged residues. These negatively supercharged variants were created to counter the high positive charge of the metals in the loaded form of the protein, where two candidates were obtained and tested, pir1_neg1 and plr1_neg2.

The used pre-release version 0.32 of the Damietta software is available at https://bio.mpg.de/damietta. The combinatorial sampler application (damietta_cs_f2m2f_v032) was used to mutate and repack the respectively indicated positons as indicated by the example input spec file used to design plr1:

```
library /tmp/global2/melgamacy/damietta_archive/damietta_v032/libv032_100
input cu3_wt_autopsf.pdb
# designable residues
mut_res 21 KREQ
mut_res 24 KREQ
mut_res 25 KREQ
mut_res 28 KREQ
mut_res 32 KREQ
mut_res 38 KREQ
mut_res 42 DKRQNSTEH
mut_res 43 DKRQNSTEH
mut_res 49 KREQ
mut_res 56 KREQ
mut_res 60 KREQ
mut_res 64 KREQ
mut_res 75 DKRQNSTEH
mut_res 78 DKRQNSTEH
mut_res 79 KREQ
mut_res 82 KREQ
mut_res 85 KREQ
mut_res 88 KREQ
mut_res 89 KREQ
mut_res 111 KREQ
mut_res 112 KREQ
mut_res 118 KREQ
# repacking residues
rpk_res 17
rpk_res 31
rpk_res 35
rpk_res 39
rpk_res 53
```

```
rpk_res 57
rpk_res 67
rpk_res 81
rpk_res 92
rpk_res 93
rpk_res 96
rpk_res 115
rpk_res 119
# sampling parameters (optional)
scramble_order 1 # default:= 0
m_mutations 3 # default:= 3
n_paths 7 # default:= 1
n_iters 5 # default:= 1
# mutagenesis scoring weights (optional)
mut_max_lj 25.0
mut_w_pp 1.0
mut_w_k 0.0
mut_w_lj 1.0
mut_w_solv 1.0
mut_w_elec 0.125
# repacking scoring weights (optional)
rpk_max_lj 25.0
rpk_w_pp 1.0
rpk_w_k 1.0
rpk_w_lj 1.0
rpk_w_solv 1.0
rpk_w_elec 0.125
```

The above input spec file was run 100 instances, for 100 CPU hours/instance.

*Purification of copper-binding proteins*

The synthetic genes for all the tested designs, and the design template were cloned without purification tags in (Table 1) in a pET28a(+) vector. The proteins were transformed and expressed in *E. coli* BL21 (DE3). Expression was induced in 2-litre LB medium at an optical density (O.D.600) of 0.8, and was done overnight at 25 C. Cells were harvested by centrifugation at 5000 g at 4°C for 20 min and lysed in 30 ml of lysis buffer (1M guanidinium chloride, 100 mM NaCl, 50mM Tris-HCl pH 8.0) supplemented with a tablet of the cOmplete, EDTA-free Protease Inhibitor Cocktail (Roche, 5056489001) and 3 mg of lyophilized DNase I (PanReac AppliChem, A3778) using a Branson Sonifier 250 (Fisher Scientific). The lysate was cleared by centrifugation at 28000 g at 4°C for 50 min and the supernatant was filtered through a 0.45 μm filter (Millipore, SLHV033RS). The sample was diluted 5-fold and applied to a 5 ml HiTrap Capto Q or S columns depending on their isoelectric point (Cytiva, 11001303, or 17544123, respectively), and eluted in a 20 mM HEPES buffer pH 7,4 using a gradient of 0 to 1.5 M KCl. The relevant fractions were identified by SDS-PAGE analysis, and further purified on a HiLoad 16/600 Superdex 200 gel filtration column (Cytiva, GE28-9893-35) using PBS. Gel filtration fractions containing pure protein in the desired oligomeric state were pooled, concentrated, and stored at -20°C for subsequent analyses.

**Table 1.** Protein sequences of template and designed copper-binding proteins

| Name | Sequence |
|------|----------|
| Csp1 | MGAKYKALLDSSSHCVAVGEDCLRHCFEMLAMNDASMGACTKATYDLV AACGALAKLAGTNSAFTPAFAKVVADVCAACKKECDKFPSIAECKACGE ACQACAEECHKVA (SEQ ID NO: 1) |
| plr1 | MGAKYKALLESSRRCVRVGERCLRHCREMLRRNDASMGACTKATYDL VKACAELAKLAGTNSARTPKKAKQVARVCEKCKKECDKFPSIAECKACA EACKKCAEECRKVA (SEQ ID NO: 3) |

(continued)

| Name | Sequence |
|------|----------|
| plr2 | MGAKYKALLRSSRRCVKVGEECLRHCREMLKRNDASMGACTKATYDLV KACARLAKLAGTNSARTPRRAKRVARVCERCKKECDKFPSIAECKACAE ACQRCAEECKKVA (SEQ ID NO: 4) |
| plr1_cr 3 | MHGAKYKALLESSRRCVRVGERCLRHAREMLRRNDASMGALTKATYDL VKACAELAKLAGTNSARTPKKAKQVARVCEKCKKECDKWPSMAEAKAC AEACKKCAEECRKVA (SEQ ID NO: 5) |
| plr1_cr 61 | MHGAKYKALLESSRRCVRVGERALRHAREMLRRNDASMGAATKAFYDL VKACAELAKLAGTNSARTPKKAKQVARVCEKCKKEADKWPSYAEAKAA AEACKKCAEECRKVA (SEQ ID NO: 6) |
| plr1_cr 62 | MHGAKYKALLESSRRCVRVGERWLRHAREMLRRNDASMGAATKAAYD LVKACAELAKLAGTNSARTPKKAKQVARVCEKCKKEADKWPSMAEAKA AAEACKKCAEECRKVA (SEQ ID NO: 7) |
| plr1_n eg1 | MHGAHYAALLESSERCVEVGERCLEHCQEMLEKNDESMGACTKATEDL VKACEELAKLAGTESAQTPELAAEVARVCEQCQKECDKFPSIEECKECA EACQECAEECEKVA (SEQ ID NO: 8) |
| plr1_n eg2 | MHGAHYEALLESSERCVEVGERCLEHCQEMLEKNDESMGACTKATEDL VKACEELAKLAGTESAQTPELAAEVARVCRQCAKECDKFPSIEECKECA EACEECAEECRKVA (SEQ ID NO: 9) |

*Estimation of Cu(II) affinity and binding capacity*

Affinity of plr1 to Cu (II) was determined using a slightly modified Zincon assay already described. The competition study of 50 $\mu$M Zincon (Supelco, 96440) with plr1 was performed in 50 mM HEPES buffer, pH 7.4, containing 100 mM NaCl. Zincon was partially saturated by the addition of Cu(II) (CuSO$_4$) to its final concentration of 20 $\mu$M. Different concentrations of plr1 ranging from 0 to 140 $\mu$M were added to the samples. The exact concentrations of Cu(II)ZI complex present in each sample were calculated based on the absorbances at 599 nM using the molar absorption coefficient of Cu(II)ZI at pH 7.4, 26100 M$^{-1}$cm$^{-1}$. Absorbances of the samples were measured on a Synergy HTX Microplate Reader (BioTek) in a 96-well plate (Greiner UV-star, 781801). The dissociation constant of plr1 ($K_d^{Plr1}$) was calculated as follows:

$$K_d^{plr1} = \frac{K_d^{Cu(II)ZI}}{K_{ex}}$$

where $K_d^{Cu(II)ZI}$ is a dissociation constant of Cu(II)ZI at pH 7.4, 4.68$\times$10$^{-17}$ M, and $K_{ex}$ is a constant describing the reaction of Cu(II) ion transfer from Cu(II)ZI to plr1. $K_{ex}$ was determined by fitting the experimental data to the following equation:

$$K_{ex} = \frac{[ZI][Cu(II)plr1]}{[Cu(II)ZI][plr1]}$$

To evaluate how many Cu(II) ions can be bound within the core of plr1, we recorded absorption spectra (from 400 nM to 700 nM) for samples containing 20 $\mu$M of Zincon, 20 $\mu$M of Cu(II) and varying concentrations of plr1 (to provide ratio Cu(II)/plr1 from 1 to 19). In case, when plr1 is saturated with Cu(II) ions, complex between Cu(II) and Zincon forms and characteristic Cu(II)ZI peak at 599 nM can be observed on the spectrum.

*Thermostability analysis*

Circular dichroism (CD) spectra were recorded using a JASCO J-810 spectrometer. Samples of plr1 and WT Csp1 (0.3 ml) at a concentration of 0.5 mg/ml of the respective proteins in 20 mM HEPES, 150 mM NaCl (pH 7.4) were loaded into 2 mm path length cuvettes. Spectral scans of mean residual ellipticity were measured at a resolution of 0.1 nm across a range of 240-195 nm. The mean residual ellipticity at a wavelength of 222 nm across a temperature range of 20-100 °C (with an increase of 1 °C/min) was tracked in melting and cooling curves. Additional thermostability analysis was performed on different proteins using nanoscale differential scanning fluorimetry (nanoDSF), which was done using Prometheus NT.48 (Nanotemper) and standard Prometheus capillaries (Nanotemper, PR-C002). The same temperature ramp parameters used for CD were set for melting and cooling, using 1 mg/ml protein samples in same buffer. In these nanoDSF experiments, fluorescence intensities at 330 nm and 350 nm, and incident beam scattering were tracked across the covered temperature range. The maxima of the first derivative of the 350 nm/330 nm ratio change were used to derive the midpoint for unfolding (i.e. melting temperature; $T_m$) and refolding during the melting and cooling ramps, respectively. Likewise, the maxima of the first derivatives of the scattering (backreflection) signal was used to identify the midpoint of colloidal aggregation (aggregation temperature; $T_{agg}$).

2. Results

Rotamer library construction

Traditionally, rotamer libraries have been constructed from amino acids conformations pooled from known structures. As the structural databases grew in size, more stringent inclusion criteria have been imposed, greatly improving the quality of available libraries. Nonetheless, PDB-based rotamer libraries can still provide a sparse coverage of the rotameric space, and entrain undesirable factors pertinent to protein structure determination such as cryogenic measurement conditions, ensemble-averaging and biased fitting. This has motivated the inventors to use molecular dynamics (MD) as a means for more extensive sampling of the conformational tendencies of amino acids in folded proteins. Furthermore, an even broader conformational distribution that is unbiased by the choice of input protein structures can be achieved through MD simulations of capped amino acids. Such conformational distributions more faithfully reproduce the tendencies of the random coil state, representing a reference energy distribution prior to any folding event. The inventors have therefore chosen to build the Damietta rotamer libraries using MD simulations of isolated amino acids (i.e., Ac-X-NHMe). The internal energies related to backbone conformational preferences were derived from occupancy of backbone bins of the dihedral space (i.e. $(\varphi, \psi)$ angles). The side chain conformational preferences were nonetheless mapped in the Cartesian space by means of RMSD clustering after alignment to a ($C^\beta$-$C^\alpha$-N) frame-of-reference. This was deliberately sought to obtain a constant number of rotamer clusters for every $(\varphi, \psi)$-bin, regardless of the number of atoms or conformational spread of the amino acid. Each cluster would be represented by a single rotamer in the final library, where the relative energy of the rotamer relates to its respective cluster size (Materials and methods).

This approach brings several advantages to the Damietta framework. First, through this approach, the internal energies derived from the conformational preferences of amino acids are consistent with the other non-bonded energy terms used in design calculations, as both rely on the same force field. Second, the rotamers generated can implicitly encode the dynamic influences on bond angles, bond stretching, and improper dihedrals. These subtle deformations are generally dismissed in protein design rotamer libraries, but have been shown to significantly impact the energy gap between native and non-native states of a protein. Third, this approach offers great scoring versatility - while here the inventors used the CHARMM force field for both rotamer library creation and the design energy function, this approach can be used to deploy more complex potentials (e.g., polarizable force fields). It also can be extended to cover rotameric distributions in different pH or sequence (e.g., tri- or penta-peptides) contexts, and can generate on-demand rotamers for non-standard amino acids or ligands. Fourth, by raising the temperature of the isolated amino acid MD simulations, broader coverage of otherwise poorly sampled $(\varphi, \psi)$-regions can be covered more effectively than in PDB-based rotamer libraries. This can be particularly successful in better accessing rare "linchpin" rotamers reported to constitute sampling bottlenecks. Lastly, the extraction of a constant number of rotamers plays an important computational role downstream, as it dictates a defined rotameric sampling granularity. This guarantees a uniform load balancing during the design calculations, particularly in parallelized solution searching applications.

*Tensorised molecular interaction fields, energies, and mechanics*

Through developing the Damietta framework, the inventors aim at achieving more efficient and more accurate design computations. Towards the efficiency end, the inventors sought to investigate two principles. The first principle is to precompute and store most of the information needed for energy calculation. The second principle is to deploy a tensorized form of the energy functions to better fit *single instruction, multiple data* processing paradigm, a hallmark of modern computing technology (Fig. 1A).

In this framework, the scoring problem is simplified to a two-body problem, where the *1st body* represents the chemical environment surrounding the side chain at the designable position, wherein the atoms of this side chain are absent. The *2nd body* represents the inbound side chain rotamer, aligned to the same frame-of-reference. The information on this two-body interaction is encoded in an asymmetric fashion in which 1st body only encodes the three-dimensional occupancy of its atoms' positions and charges, while the 2nd body encodes the net, real-valued energy field around all of its respective atoms (Fig. 1B). Indeed, the computationally expensive step is the projection of energy fields, which in this case is restricted to the *2nd body* (i.e., the rotamer), and is hence precomputed once and stored in a lookup table. This restricts the runtime computing load to simply mapping the *1st body* three-dimensional occupancy; substantially reducing the runtime needed at every designable position. Such a representation benefits from further speedup when implemented in a tensorized fashion. In this manner, the scalar-valued interaction energy between the two bodies is obtained by sum of the element-wise product of the two tensors (representing environment and the rotamer energy field). This format is ideally suited for evaluating both the LJ and electrostatic potentials, albeit at the cost of assuming symmetric LJ parameters for the interacting atom pairs, according to the atom's respective encoding in the *2nd body* tensor. Applying the tensorization framework would differ however in the case of encoding a surface area-based solvation potential. In this situation the *1st body* tensor has to fully describe environment's surface solvation energy field. Hence, for solvation, the solvation energy per unit surface area will be normalised by the number of voxels representing the atomic surface (Methods section). Such a tensor is precomputed for the 2nd body (i.e. the inbound side chain rotamer), is computed on-the-fly for the *1st body,* as well as for the combined two bodies (Materials section). This renders the solvation term the most expensive energy term to compute.

*Scoring function accuracy*

The inventors sought to evaluate the accuracy of the Damietta energy function to predict the impact of single-point mutation away from added complexities of combinatorial repacking and design. The Damietta energy values were evaluated without any combinatorial side chain optimization, but by finding the lowest energy rotamer at the designated position, and evaluating the energy difference between the mutant and wild type (Materials and methods). The inventors started by dataset of Gβ1 mutants which constitutes the largest thermodynamic stability dataset collected in a single experimental setup. This dataset covers almost the entire single-point mutagenesis landscape of the Gβ1 protein, and represents a broad range of burial and secondary structure contexts. In this setup, $\Delta\Delta G$ values obtained by the Damietta potential showed a slightly better correlation ($R = 0.35$, $p = 2.5 \times$ ) compared to the reported Rosetta score ( $R = 0.32$, $p = 1.8 \times$ ) (Fig. 2A). The Gβ1 dataset however has indicated a clear bias for hydrophobics. Therefore, the inventors further evaluated the energy function accuracy against other datasets that contain more polar residues that are either buried or solvent-exposed. The second dataset comprises mutations at and around the active site of β-glucosidase B (PDB ID: 2JIE), where the predicted free energy change showed clearly better correlations with experimental $\Delta\Delta G$ ( $R = 0.34$, $p = 1.4 \times$ ; Fig. 2B), $\Delta\Delta H$, and $\Delta\Delta S$ values (Fig. 3) than those predicted by the Rosetta scoring function. The third and electrostatics-focused dataset was comprised of charge-reversing or charge-neutralising mutations, in 4 different proteins; T4 lysozyme (PDB ID: 3LZM), human lysozyme (PDB ID: 1 REX), ribonuclease Sa (PDB ID: 1C54), and cold shock protein B (PDB ID: 1CSP). Despite the minimal hydrophobic contribution of these mutations, the overall correlation coefficient with the experimental $\Delta\Delta G$ value was similar to the other datasets ($R = 0.31$, $p = 1.7 \times$ , Fig. 2C), which highlights the generality of the energy function.

*Combinatorial design by decision tree swarm*

The *highly dimensional nature* of *combinatorial* design of more than a few amino acid positions can severely limits the practicality of exact sampling algorithms in finding global minimum solutions within reasonable computing timeframes. Nonetheless, despite the non-additive effects of multiple correlated mutations, favorable mutations generally tend to cluster closely in the sequence space. Thus, a swarm of greedy samplers traversing many sequence optimization paths can generally reduce entrapment within local minima and lead to near-optimal solutions, which is clearly demonstrated by the success of stochastic design algorithms. In order to enable the exploration of a sizable number of mutations, we have developed a combinatorial sampling strategy that searches for successive minima by spanning a few loosely communicating independent search paths. This strategy builds on the power of parallel, loosely-communicating conformational samplers that assume a smooth, but locally rugged, landscape as was demonstrated with the SARS and FLAPS algorithms. One way of implementing this approach in a design context is through a *few-to-many, many-to-few* (i.e. f2m2f) scheme, whereby designable amino acid positions are arranged as depth levels within a tree, and nodes at each level represent the target mutations. As the branching represents the expansion of mutant combinations (i.e. *many* combinatorial candidates), a ranking-and-trimming step only keeping the few lowest energy designs is imposed between the layers of the tree (Fig. 4). This alternation between the *many* combinations and the *few* best guarantees that a constant number of branches is being traversed down the tree depth at any given level, restraining the combinational load complexity, and providing an ideal parallelization scheme.

In conventional stochastic sampling algorithms, higher energy mutations can still be accepted at a lower probability in favor of basin hopping and diversity generation. However, this equates to sowing randomness over the existing heterogeneous scoring function uncertainty (i.e., scoring error). Therefore, instead of, for example, a Metropolis criterion, and since sampling a large number of mutations can never be exhaustive, design simulation replicas with randomly ordered designable positions along the decision tree can generate diversity. This was implemented in the combinatorial sampler by running independent instances of the design simulation, which in addition to the inclusion of the lowest energy $m$ mutations at each position enable exploring multiple optimization paths and allow basin hopping (Fig. 4). This implementation further allows specifying the number of iterated traversals of the same decision tree in order improve the search convergence (Fig. 4). The synthetic genes encoding the designs, or Csp1 were cloned without purification tags in a vector for expression in *E. coli.* The soluble expression levels were highest for plr1, followed by plr2, both being higher than the expression level of the template. In contrast to the template, which has a net negative charge of -2, the designs possessed high net-positive charges (plr1: +14; plr2: +17, plr1_cr3: +14; plr1_cr61: +14; plr1_cr62: +14) or net-negative charge (plr1_neg1: -16; plr1_neg2: -14). This greatly facilitated the purification of the designs using cation exchange chromatography (whereas the template was purified using anion exchange chromatography). The purification yield was the highest for plr1 (>50 mg per liter-culture), therefore, the inventors demonstrate the following experimental characterization to the plr1 design.

The inventors set out to evaluate the oligomeric state, thermostability, and metal binding properties of their design. Analytical size exclusion showed plr1 to be purely monomeric, in contrast to the template Csp1 which was tetrameric and showed significant sample degradation (Fig 5A, B). Circular dichroism (CD) spectroscopy showed the apo-protein form of plr1 to be strongly helical with a melting mid-point temperature $T_m = 67$ °C. This thermal unfolding was reversible with no observed precipitation, where the sample recovered most of its initial residual ellipticity (Fig 5C, D). Equilibrium fitting indicated an unfolding free energy of $\Delta G_{unfold} = 48.3$ *kJ/mol* at 20 °C, highlighting the stability of the plr1 design. The melting ($T_m$) and aggregation temperatures ($T_{agg}$) using nanoDSF are summarized in the following:

WT Csp1:

$$T_m = 79.1 \pm 0.06 \ ^\circ C$$

$$T_{agg} = 59.42 \pm 0.13 \ ^\circ C$$

Irreversible unfolding due to aggregation.
plr1:

$$T_m = 67.9 \pm 0.05 \ ^\circ C$$

$$T_{agg} > 100 \ ^\circ C$$

Reversible folding
plr1_cr3:

$$T_m = 60.56 \pm 0.28 \ ^\circ C$$

$$T_{agg} > 100 \ ^\circ C$$

Reversible folding
plr1_cr61 :

$$T_m = 60.09 \pm 0.03 \ ^\circ C$$

$$T_{agg} > 100 \ ^\circ C$$

Reversible folding
plr1_cr62:

$$T_m = 58.62 \pm 0.05 \ ^\circ C$$

$$T_{agg} > 100 \ ^\circ C$$

Reversible folding
plr1_neg2:

$$T_m = 54.52 \pm 0.18 \ ^\circ C$$

$$T_{agg} > 100 \ ^\circ C$$

Reversible folding.

To evaluate the Cu(II) binding affinity, the inventors used a competitive binding assay with Zincon (ZI). Zincon is a chromophoric probe with ultra-high affinity for Cu(II) and other transition metal ions, which is reliably used in metal detection and metal affinity assays. Competitive binding titrations showed a 1:1.3 [ZI]:[plr1] titration mid-point for Cu(II) binding, indicative of a dissociation constant $K_d = 7.8 \times 10^{-17} M$ for plr1 (Fig. 5E). Such an ultra-tight binding is expected to be very favourable for imaging applications. To further quantify the number of metal binding sites on plr1, the inventors conducted UV/Vis spectral scans at constant concentrations of Zincon and Cu(II), and varying concentrations of plr1. The mid-point of chromophoric change was clearly observed between 12:1 and 13:1 [Cu(II)]:[plr1] ratio, indicating an average 12.5 Cu(II) binding sites on plr1 (Fig. 5F). This high metal binding capacity of almost 1 metal/1 kDa of protein would be highly beneficial in metal recovery applications, as well as for high imaging sensitivity and efficacy in the case of radiotracer imaging and radioimmunotherapy applications, respectively.

3. <u>Conclusions and outlook</u>

The inventors used Damietta to reengineer a natural copper binder. Redesigned copper-binder, in turn, is stable, purely monomeric and can be purified in much higher yields compared to the native template, that might be advantageous in various applications, from metal decontamination to radio-imaging.

**Claims**

1.  A polypeptide comprising an amino acid sequence having at least 60% and at most approx. 98% homology with the amino acid sequence of copper storage protein from *Methylosinus trichosporium* OB3b (Csp1).

2.  The polypeptide of claim 1, which is configured to assemble into a monomeric metal binding protein, preferably a transition metal binding protein, further preferably said transition metal is selected from Cu(II), Pb(II), and Co(II).

3.  The polypeptide of any of the preceding claims, wherein the amino acid sequence of which comprising at most approx. 95%, preferably at most approx. 90%, further preferably at most approx. 85, and highly preferably approx. 81% homology with the amino acid sequence of Csp1 (SEQ ID NO: 1).

4.  The polypeptide of any of the preceding claims, which comprises the following amino acid sequence SEQ ID NO: 2:

$$X_1X_2GAX_3YX_4ALLX_5SSX_6X_7CVX_8VGEX_9X_{10}LX_{11}HX_{12}X_{13}EMLX_{14}X_{15}NDX_{16}SMGAX_{17}TKAX_{18}X_{19}DLVX_{20}ACX_{21}X_{22}LAKLAGTX_{23}SAX_{24}TPX_{25}X_{26}AX_{27}X_{28}VAX_{29}VCX_{30}X_{31}CX_{32}KEX_{33}DKX_{34}PSX_{35}X_{36}EX_{37}KX_{38}X_{39}X_{40}EACX_{41}X_{42}CAEECX_{43}KVA,$$

wherein:

$X_1$ = M or missing,
$X_2$ = M or H,
$X_3$ = K or H,
$X_4$ = K, A or E,
$X_5$ = D, E or R,
$X_6$ = S, R or E,
$X_7$ = H or R,
$X_8$ = A, R or K,
$X_9$ = D, R or E,
$X_{10}$ = C, A or W,
$X_{11}$ = R or E,
$X_{12}$ = C or A,
$X_{13}$ = F, R or Q,
$X_{14}$ = A, R, K or E,
$X_{15}$ = M, R or K,
$X_{16}$ =A or E,
$X_{17}$ =C, L or A,
$X_{18}$ =T, F or A,
$X_{19}$ =Y or E,
$X_{20}$ =A or K,
$X_{21}$ =G, A or E,
$X_{22}$ =A, E or R,
$X_{23}$ =N or E,
$X_{24}$ =F, R or Q,
$X_{25}$ =A, K, R or E,
$X_{26}$ =F, K, R or L,
$X_{27}$ =K or A,
$X_{28}$ =V, Q, R or E,
$X_{29}$ =D or R,
$X_{30}$ =A, E or R,
$X_{31}$ =A, K, R or Q,
$X_{32}$ =K, Q or A,
$X_{33}$ =C or A,
$X_{34}$ =F or W,
$X_{35}$ =I, M or Y,
$X_{36}$ =A or E,
$X_{37}$ =C or A,
$X_{38}$ =A or E,
$X_{39}$ =C or A,
$X_{40}$ =G or A,
$X_{41}$ =Q, K or E,
$X_{42}$ =A, K, R or E,
$X_{43}$ =H, R, K or E.

5. The polypeptide of any of the preceding claims, which comprises any of the following amino acid sequences: SEQ ID NO: 3 (plr1), SEQ ID NO: 4 (plr2), SEQ ID NO: 5 (plr1_cr3), SEQ ID NO: 6 (plr1_cr61), SEQ ID NO:7 (plr1_cr62), SEQ ID NO:8 (plr1_neg1), SEQ ID NO:9 (plr1_neg2).

6. A protein comprising:

   a) a single polypeptide chain derived from copper storage protein from *Methylosinus trichosporium* OB3b (Csp1);
   b) a bundle of four amphiphatic $\alpha$-helices located on said single polypeptide chain;
   c) three amino acid linkers that connect contiguous bundle-forming $\alpha$-helices;

   wherein the protein comprises at least one metal binding site.

7. The protein of claim 8, wherein it has a melting temperature ($T_m$) of at least $\geq 50°C$, preferably of at least $\geq 100°C$, and/or

   wherein it has an aggregation temperature ($T_{agg}$) of at least $\geq 50°C$, preferably of at least $\geq 100°C$, and/or it binds metal with a dissociation constant ($K_D$) of at least $\leq 1fM$, preferably of at least $\leq 1$ fM.

8. The protein of claim 6 or 7, **characterized in that** each amino acid linker has a length between 2 and 20, preferably between 2 and 15, more preferably between 2 and 10, and most preferably between 3 and 7 amino acids.

9. The protein of any of claims 6-8, **characterized in that** each $\alpha$-helix comprises one or more cysteine residue(s), preferably the frequency of cysteine residues along an $\alpha$-helix sequence is at least one cysteine is located at every third position $(XXC)_n$, and at most one cysteine at every 11th position $(XXXXXXXXXXC)_n$.

10. The protein of any of claims 6-9, **characterized in that** the single polypeptide chain is the polypeptide of any of claims 1-5.

11. A nucleic acid molecule encoding the polypeptide of any of claims 1-5 or the protein of any of claims 6-10, optionally linked to a promoter sequence.

12. A recombinant host cell comprising the polypeptide according to any of claims 1-5, or the protein of any of claims 6-10, or the nucleic acid of claim 11.

13. A pharmaceutical composition cell comprising the polypeptide according to any of claims 1-5, or the protein of any of claims 6-10, the nucleic acid of claim 11, or the recombinant host cell of claim 12, preferably said pharmaceutical composition is selected from the group consisting of: radio immunotherapeutic agent, radio tracing agent, contrast agent, antidot for metal intoxication, metal-decontamination agent, and metal recovery agent.

14. A use of the polypeptide according to any of claims 1-7 for reconstituting a monomeric metal binding protein, preferably a transition metal binding protein.

15. A copper storage protein from *Methylosinus trichosporium* OB3b (Csp1) and/or a polypeptide comprising the amino acid sequence of SEQ ID NO: 1 for use as a medicament, preferably selected from the group consisting of: radio immunotherapeutic agent, radio tracing agent, contrast agent, antidot for metal intoxication, metal-decontamination agent, and metal recovery agent.

**Fig. 1**

**A)**

| CPU | CPU | GPU | HPC |
|---|---|---|---|
| Reference performance | >200-fold speedup | >6000-fold speedup | Same tensor size Load-balanced |

**B)** Target position

**C)** Delete side chain

**D)**

Map *environment tensor*    Inbound rotamer *field tensor*

Define tensor frame

$\odot$

= Yield interaction energy

# Fig. 2

## A)

## B)

## C)

# Fig. 3

# Fig. 4

```
                    ┌──────────────┐
                    │    Input     │
                    │  Structure   │
                    └──────────────┘
                           │
                           ▽
        Yes          ╱ Scramble ╲          No
      ┌──────────────   order?   ──────────────┐
      │              ╲           ╱             │
      ▽                                        ▽
┌──────────────┐                        ┌──────────────┐
│Sort mutable  │                        │Sort mutable  │
│residues      │                        │residues      │
│randomly      │                        │by energy     │
└──────────────┘                        └──────────────┘
```

Mutate current position

Repack all mutable and designable positions of mutant *1*

Repack all mutable and designable positions of mutant *2*

Repack all mutable and designable positions of mutant *N*

Pick lowest energy *m* mutants

$m > n_{paths}$

No — Keep all mutant combinations

Yes — Keep lowest energy $n_{path}$ mutants

Yes

If *iterations* < $n_{Iter}$ trim to $n_{paths}$

No → End

## Fig. 5

**EUROPEAN SEARCH REPORT**

| | Application Number |
|---|---|
| | EP 22 20 6059 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VITA NICOLAS ET AL: "A four-helix bundle stores copper for methane oxidation", NATURE, vol. 525, no. 7567, 1 September 2015 (2015-09-01), pages 140-143, XP093043136, London ISSN: 0028-0836, DOI: 10.1038/nature14854 Retrieved from the Internet: URL:https://www.ncbi.nlm.nih.gov/pmc/articles/PMC4561512/pdf/emss-63964.pdf> | 1-3,6-12 | INV. A61P43/00 C07K14/195 |
| Y | * abstract * <br> * page 4, paragraph 3 * <br> * page 13; figure 1 * <br> * page 15; figure 3 * <br> ----- | 4 | |
| X | US 11 471 497 B1 (BERMUDES DAVID GORDON [US]) 18 October 2022 (2022-10-18) * abstract * * column 34; claim 1; figure 3 * ----- | 1-3,6-15 | |
| X | LOMBARDI ANGELA: "Simple structure, complex function", NATURE CHEMICAL BIOLOGY, vol. 11, no. 10, 1 October 2015 (2015-10-01), pages 760-761, XP093043124, New York ISSN: 1552-4450, DOI: 10.1038/nchembio.1918 Retrieved from the Internet: URL:http://www.nature.com/articles/nchembio.1918> * the whole document * ----- <br> -/-- | 1-3,6 | TECHNICAL FIELDS SEARCHED (IPC) <br><br> A61P C12R C07K |

The present search report has been drawn up for all claims

| Place of search | Date of completion of the search | Examiner |
|---|---|---|
| The Hague | 7 June 2023 | Gurdjian, Didier |

Europäisches
Patentamt

European
Patent Office

Office européen
des brevets

## EUROPEAN SEARCH REPORT

Application Number

EP 22 20 6059

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE UniProt [Online]<br><br>12 October 2022 (2022-10-12),<br>"RecName: Full=Four-helix bundle copper-binding protein {ECO:0000256\|Google:ProtNLM};",<br>XP002809156,<br>retrieved from EBI accession no. UNIPROT:A0A1A6FPL1<br>Database accession no. A0A1A6FPL1<br>* the whole document * | 4 | |

TECHNICAL FIELDS
SEARCHED (IPC)

The present search report has been drawn up for all claims

| Place of search | Date of completion of the search | Examiner |
|---|---|---|
| The Hague | 7 June 2023 | Gurdjian, Didier |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 20 6059

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2023

| Patent document cited in search report | Publication date | Patent family member(s) | Publication date |
|---|---|---|---|
| US 11471497 B1 | 18-10-2022 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MALMSTROM ; NEILANDS.** Metalloproteins. *Annual Review of Biochemistry,* 1964, vol. 33 (1), 331-354
- **LU et al.** Design of functional metalloproteins. *Nature,* 2009, vol. 460 (7257), 855-862
- **CHALKLEY et al.** De novo metalloprotein design. *Nature Reviews Chemistry,* 2022, vol. 6 (1), 31-50
- **ELLISMAN et al.** Picking faces out of a crowd: genetic labels for identification of proteins in correlated light and electron microscopy imaging. *Methods in cell biology,* 2012, vol. 111, 139-155
- **MATSUMOTO ; JASANOFF.** Metalloprotein-based MRI probes. *FEBS letters,* 2013, vol. 587 (8), 1021-1029
- **SAWYER et al.** Metal-binding chimeric antibodies expressed in Escherichia coli. *Proceedings of the National Academy of Sciences,* 1992, vol. 89 (20), 9754-9758
- **NEEDLEMAN ; WUNSCH.** *J. Mol. Biol.,* 1970, vol. 48, 443-453
- **RICE, P. ; LONGDEN, I. ; BLEASBY, A.** EMBOSS: The European Molecular Biology Open Software Suite. *Trends in Genetics,* 2000, vol. 16 (6), 276-277
- **KAMTEKAR ; HECHT.** The four-helix bundle: what determines a fold?. *FASEB,* 1995, vol. 11 (11), 1013-1022
- **MCGUFFIN et al.** The PSIPRED protein structure prediction server. *Bioinformatics,* 2000, vol. 16 (4), 404-405
- **YANG et al.** SPIDER2: A package to predict secondary structure, accessible surface area, and main-chain torsional angles by deep neural networks. *PSSPred,* 2016, https://zhanglab.ccmb.med.umich.edu/PSSpred
- **WANG et al.** Protein secondary structure prediction using deep convolutional neural fields. *Scientific Reports,* 2016, vol. 6, 18962
- **SKOKOWA et al.** *Nat Commun,* 2022, vol. 13, 2948